# EUROPEAN PATENT SPECIFICATION

(11) **EP 1 917 017 B1**
(45) Date of publication and mention of the grant of the patent: **03.02.2010**
(21) Application number: 06736552.8
(22) Date of filing: 01.03.2006
(51) Int. Cl.: A61K 31/505, A61P 25/28

(54) **TREATMENT OF AMYOTROPHIC LATERAL SCLEROSIS WITH PYRIMETHAMINE AND ANALOGUES**
BEHANDLUNG VON AMYOTROPHISCHER LATERALSKLEROSE MIT PYRIMETHAMIN UND ANALOGA
TRAITEMENT DE LA SCLEROSE AMYOTROPHIQUE LATERALE AVEC LA PYRIMETHAMINE ET DES ANALOGUES

(30) Priority: 04.03.2005 US 658505 P
(43) Date of publication of application: 07.05.2008
(73) Proprietor: Alsgen, Inc., Monmouth Junction, NJ 08852 (US)
(72) Inventor: SCOTT, Sean, San Francisco, California 94132 (US); BENJAMIN, Daniel, Englishtown, New Jersey 07726 (US)
(74) Representative: Hiebl, Inge Elisabeth
(86) International application number: PCT/US2006/007257
(87) International publication number: WO 2006/096405

(56) References cited:
- EP-A1- 1 473 289
- WO-A-2006/096404
- ANONYMOUS: "CID 4993: Listing of all related compounds of pyrimethamine in PubChem." PUBCHEM COMPOUND, [Online] XP002418178 Retrieved from the Internet: URL:http://www.ncbi.nlm.nih.gov/entrez/que ry.fcgi?CMD=Display&DB=pccompound>
- OKUNO TATSUSADA ET AL: "Induction of cyclooxygenase-2 in reactive glial cells by the CD40 pathway: relevance to amyotrophic lateral sclerosis" JOURNAL OF NEUROCHEMISTRY, vol. 91, no. 2, October 2004 (2004-10), pages 404-412, XP002418174 ISSN: 0022-3042
- ROTHSTEIN JEFFREY D ET AL: "Beta-lactam antibiotics offer neuroprotection by increasing glutamate transporter expression." NATURE 6 JAN 2005, vol. 433, no. 7021, 6 January 2005 (2005-01-06), pages 73-77, XP002418177 ISSN: 1476-4687

## Description

### Background of the Invention

Amyotrophic lateral sclerosis (ALS) is the most commonly diagnosed progressive motor neuron disease. The disease is characterized by degeneration of motor neurons in the cortex, brainstem and spinal cord (Principles of Internal Medicine, 1991 McGraw-Hill, Inc., New York; Tandan et al. (1985) Ann. Neurol, 18:271-280, 419-431). The cause of the disease is unknown and ALS may only be diagnosed when the patient begins to experience asymmetric limb weakness and fatigue, localized fasciculation in the upper limbs and/or spasticity in the legs which typifies onset. There is a genetic component to at least some incidences of ALS.

In almost all instances, sporadic ALS and autosomal dominant familial ALS (FALS) are clinically similar (Mulder et al. (1986) Neurology, 36:511-517). It has been shown that in some but not all FALS pedigrees the disease is linked to a genetic defect on chromosome 21q (Siddique et al., (1991) New Engl. J. Med., 324:1381-1384).

In particular, mutations in the SOD-1 gene which is localized on chromosome 21q, appear to be associated with the familial form of ALS. The deleterious effects of various mutations on SOD-1 are most likely mediated through a gain of toxic function rather than a loss of SOD-1 activity (Al-Chalabi and Leigh, (2000) Curr. Opin. Neurol., 13, 397-405; Alisky et al. (2000) Hum. Gene Ther., 11, 2315-2329). While the toxicity is unclear, there exists evidence to suggest that elimination of the protein itself will ameliorate the toxicity.

A need exists to develop therapies that can alter the course of neurodegenerative diseases or prolong the survival time of patients with such diseases. In particular, a need exists to reduce the SOD-1 protein produced in the brain and spinal cord of ALS patients. Preventing the formation of wild type or mutant SOD-1 protein may stop disease progression and allow for amelioration of ALS symptoms.

EP 1 473 289 discloses pyrimethamine analogs to treat Alzheimer disease.

### Summary of the Invention

Uses are disclosed for interfering with protein synthesis in the brain, spinal cord, meningia and muscle cells by administrating a pyrimethamine or a functional analog thereof. In particular, a pyrimethamine or a functional analog thereof for use in decreasing SOD-1 gene expression is disclosed.

The use of the invention can be used to reduce or inhibit the expression of a protein associated with SOD-1-mediated amyotrophic lateral sclerosis, being SOD-1. The use of pyrimethamine or a functional analogue thereof inhibits SOD-1 mRNA transcription or the stability of the transcript. The decreases in SOD-1 mRNA then lead to decreased protein levels of SOD-1, which reduce its accumulation in the cell and ameliorate the disease. The expression and accumulation of mutant SOD-1 is a widely accepted pathophysiological mechanism underlying familial ALS, and might also play a role in the sporadic form of the disease.

Accordingly, the invention pertains to the use of a pyrimethamine or functional analog thereof for the manufacture of a medicament for preventing the development of symptoms, or ameliorating the symptoms or progression of SOD-1-mediated amyotrophic lateral sclerosis (ALS). The cell being the target of said use can be a neural cell, or any cell in the spinal cord, the meningial tissue, or a muscle cell, for example in a subject with ALS (e.g., familial ALS). Examples of cells include, but are not limited to, neurons, intemeurons, glial cells, microglia cells, muscle cells, cells involved in the immune response.

The invention further relates to a pyrimethamine or a functional analog thereof for use in a method for preventing the development of symptoms, or ameliorating the symptoms or progression of SOD-1-mediated amyotrophic lateral sclerosis (ALS).

In one embodiment, pyrimethamine or functional analogs thereof are used. In another embodiment, pyrimethamine with at least one modification in the benzene ring is used. In yet another embodiment, pyrimethamine with at least one modification in the pyrimidine ring is used.

The inhibition of transcription of the gene comprises monitoring by measuring the expression levels of the the SOD-1 protein. Alternatively, the inhibition of transcription of the gene comprises monitoring the levels of a nucleic acid molecule that encodes the SOD-1 protein, for example by monitoring the ribonucleic acid or deoxynucleic acid levels.

The ameliorating of symptoms can be monitored by measuring the survival prolongation of the subject, for example by monitoring a neurological score of the subject. alternatively, the amelioration can be determined by monitoring the expression levels of the SOD-1 protein or the levels of a nucleic acid molecule that encodes SOD-1 protein.

### Brief Description of Drawings

Fig. 1 is a graph showing the reduction of SOD-1 protein expression by pyrimethamine.
Fig. 2 is a bar graph showing the reduced expression of SOD-1 mRNA with pyrimethamine and norethindrone.
Fig. 3 is a schematic showing a few representative pyrimethamine functional analogs of the invention.
Fig. 4 is a bar graph showing reduced expression of mRNA for alpha synuclein in HeLa cells following treatment with pyrimethamine and norethindrone.
Fig. 5 is a bar graph showing the reduction of SOD-1 protein expression in male and female SOD-93A mice with chronic pyrimethamine treatment (TX).
Fig. 6 is a bar graph showing the decrease in expression of alpha synuclein in mouse lymphocytes with chronic pyrimethamine treatment.
Fig. 7 is a bar graph showing the decreased expression of spinal SOD-1 in SOD-93A mice following oral administration of pyrimethamine.
Fig. 8 is a bar graph showing a decrease in lymphocyte SOD-1 levels in a familial SOD-1 patient following 30 days of oral administration of pyrimethamine.

### Detailed Description

The practice of the present invention employs, unless otherwise indicated, conventional methods of microbiology, molecular biology and recombinant DNA techniques within the skill of the art. Such techniques are explained fully in the literature. (*See, e.g.*, Sambrook, et al. Molecular Cloning: A Laboratory Manual (Current Edition); DNA Cloning: A Practical Approach, Vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, vol. I & II (P. Tijessen, ed.); Fundamental Virology, 2nd Edition, Vol. I & II (B. N. Fields and D. M Knipe, eds.)).

So that the invention is more clearly understood, the following terms are defined:

The term "neurodegenerative disorder" or "neurodegenerative disease" are used interchangeably herein and refer to an impairment or absence of a normal neurological function, or presence of an abnormal neurological function in a subject, or group of subjects. For example, neurological disorders can be the result of disease, injury, and/or aging. As used herein, neurodegenerative disorder also includes neurodegeneration which causes morphological and/or functional abnormality of a neural cell or a population of neural cells. Non-limiting examples of morphological and functional abnormalities include physical deterioration and/or death of neural cells, abnormal growth patterns of neural cells, abnormalities in the physical connection between neural cells, under- or over production of a substance or substances, *e.g.*, a neurotransmitter, by neural cells, failure of neural cells to produce a substance or substances which it normally produces, production of substances, *e.g.*, neurotransmitters, and/or transmission of electrical impulses in abnormal patterns or at abnormal times. Neurodegeneration can occur in any area of the brain of a subject and is seen with many disorders including, for example, Amyotrophic Lateral Sclerosis (ALS), multiple sclerosis, Huntington's disease, Parkinson's disease, Alzheimer's disease, prion associated disease (CJD), spinal muscular atrophy, spinal cerebellar ataxia, and spinal cord injury.

The terms "pharmacological agent" and "nuclear receptor modulating pharmacological agent" as used herein, are intended to be used interchangeably, and these terms refer to the compound, or compounds, that interfere selectively with protein synthesis in a neural spinal cord, menengial, or muscle cell. In particular, interfere with protein synthesis of a SOD-1 protein.

The terms "modulate" or "modulating" or "modulated" are used interchangeably herein also refer to a change SOD-1 activity, or the expression, i.e., an increase or decrease in SOD-1 activity, or expression, such that the modulation produces a therapeutic effect in a subject, or group of subjects. A therapeutic effect is one that results in an amelioration in the symptoms, or progression of ALS. The change in activity can be measured by quantitative or qualitative measurements of the SOD-1 protein level for example by Western blot analysis. The quantitative assay can be used to measure downregulation or upregulation of SOD-1 protein levels in the presence of a pharmacological agent, such as pyrimethamine and analogs thereof. A suitable pharmacological agent can be one that down-regulates SOD-1 expression by about 5 percent to about 50 percent compared with a control. The change in expression can also be measured by quantitative or qualitative measurements of the nucleic acid level associated with SOD-1, for example by measuring the expression level ofRNA or DNA.

The effect of SOD-1 modulation on a subject, or group of subjects, can also be investigated by examining the survival of the subject, or group of subjects. For example, by measuring the change in the survival, or the prolongation of survival in one or more animal models for a neurodegenerative disease, e.g., ALS. The change in the survival can be due to the administration of pharmacological agent being pyrimethamine or functional analog that is administered to an ALS murine model. The effect of the pharmacological agent can be determined based on the increase in days of survival of a test group of ALS mice compared with a control group of ALS mice that have been given a control agent, or no agent. In one embodiment, the pharmacological agent being pyrimethamine or a functional analog increases the percentage effect on survival of the subject, or a population of subjects (e.g., a male population, or a female population) by at least 2% to about 100%. Preferably the percentage effect on survival of the subject, or a population of subjects, is by at least 5% to about 50%, by at least 10% to about 25%. Even more preferably, the percentage effect on survival of the subject, or a population of subjects, is by at least 3%, 4%, 5%, 6%, 7%, 8%, 9%, 10%, 12%, 14%, 16%, 18%, 20%, 22%, 24%, 26% 28%, 30%, 32%, 34%, 36%, 38%, 40%, 42%, 44%, 46%, 48% and 50%. The effect of SOD-1 modulation may also determined by examining the neurological score of a subject, or group of subjects for example, by assessing the improvement in muscular movement, or by examining the alleviation or amelioration of the disease symptoms. In a preferred embodiment, the neurological score of a subject, or group of subjects is significantly different from that of the untreated control subjects, with a level of significance between p<0.05 and p<0.0001, as determined using standard statistical analysis procedures.

The terms may also be used to refer to a change in the nuclear receptor upon interaction with a pharmacological agent, i.e., a change in nuclear receptor activity, structure, or the expression of a nuclear receptor, or a subunit of the nuclear receptor, i.e., an increase or decrease in nuclear receptor activity, or expression, such that the modulation produces a therapeutic effect in a subject, or group of subjects.

The term "inhibit" or "inhabiting" as used herein refers to a measurable reduction of expression of a target gene or a target protein, e.g., SOD-1. The term also refers to a measurable reduction in the activity of a target protein. Preferably a reduction in expression is at least about 10%. More preferably the reduction of expression is about 20%, 30%, 40%, 50%, 60%, 80%, 90% and even more preferably, about 100%.

The SOD-1 protein can be a wild type SOD-1 protein or a mutant SOD-1 protein and can be derived from a wild type SOD-1 gene or a SOD-1 gene with at least one mutation.

The term "subject" as used herein refers to any living organism in which an immune response is elicited. The term subject includes, but is not limited to, humans, nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

As used herein, "alkyl" groups include saturated hydrocarbons having one or more carbon atoms, including straight-chain alkyl groups (*e.g.*, methyl, ethyl, propyl, butyl, pentyl, hexyl, heptyl, octyl, nonyl, decyl, etc.), cyclic alkyl groups (or "cycloalkyl" or "alicyclic" or "carbocyclic" groups) (*e.g.*, cyclopropyl, cyclopentyl, cyclohexyl, cycloheptyl, cyclooctyl, etc.), branched-chain alkyl groups (isopropyl, *tert*-butyl, *sec*-butyl, isobutyl, etc.). Unless otherwise specified the term alkyl includes both "unsubstituted alkyls" and "substituted alkyls," the latter of which refers to alkyl groups having substituents replacing one or more hydrogens on one or more carbons of the hydrocarbon backbone.

The term "alkoxy group" as used herein means an alkyl group having an oxygen atom attached thereto. Representative alkoxy groups include groups having 1-10 carbon atoms, preferably 1-6 carbon atoms, *e.g.*, methoxy, ethoxy, propoxy, *tert*-butoxy, and the like. Examples of alkoxy groups include methoxy, ethoxy, propoxy, *iso*-propoxy, butoxy, pentoxy.

The term "aromatic group" or "aryl group" includes unsaturated and aromatic cyclic hydrocarbons as well as unsaturated and aromatic heterocycles containing one or more rings. Aryl groups may also be fused or bridged with alicyclic or heterocyclic rings that are not aromatic so as to form a polycycle (*e.g.*, tetralin). Aryl groups can also be fused or bridged with alicyclic or heterocyclic rings which are not aromatic so as to form a polycycle (*e.g.*, tetralin).

An "arylalkyl" group is an alkyl group substituted with an aryl group (*e.g.*, phenylmethyl (*i.e.*, benzyl)). An "alkylaryl" moiety is an aryl group substituted with an alkyl group (*e.g., p*-methylphenyl (*i.e., p*-tolyl)). An "alkoxyphenyl" group (or "alkyloxyphenyl" group) is a phenyl group substituted with an alkoxy group (*e.g.*, *p-*methoxyphenyl). An "arylalkoxy" group is an alkoxy group substituted with a phenyl group (*e.g.*, benzyloxy), An "aryloxyalkyl" group is an alkyl group substituted with an oxyaryl group (*e.g*., phenylmethyl ether (*i.e.*, phenoxymethyl)), An "aryloxyphenyl" group is an phenyl group substituted with a phenoxy group (*e.g.,* biphenyl ether (*i.e.,* phenoxyphenyl)), A "phenoxy" group is an oxygen atom attached to a phenyl group.

The term "heteroaryl group" includes unsaturated and aromatic cyclic groups in which one or more of the carbon atoms in the ring is an element other than carbon, for example, nitrogen, sulfur, or oxygen.

The term "heterocyclic group" includes closed ring structures analogous to carbocyclic groups in which one or more of the carbon atoms in the ring is an element other than carbon, for example, nitrogen, sulfur, or oxygen. Heterocyclic groups may be saturated or unsaturated. Additionally, heterocyclic groups (such as pyrrolyl, pyridyl, isoquinolyl, quinolyl, purinyl, and furyl) may have aromatic character, in which case they may be referred to as "heteroaryl" or "heteroaromatic" groups.

An "heteroarylalkyl" group is an alkyl group substituted with a heteroaryl group (*e.g*., 4-methylpyridine).

An "heteroarylalkoxy" group is an alkoxy group substituted with a heteroaryl group (*e.g.,* 4-methoxypyridine).

### I. Neurodegenerative Diseases

Amyotrophic Lateral Sclerosis (ALS), also called Lou Gehrig's disease, is a fatal neurodegenerative disease affecting motor neurons of the cortex, brain stem and spinal cord. (Hirano, (1996) Neurology, 47(4 Suppl. 2): S63-6). Onset of ALS occurs in the fourth or fifth decade of life (median age of onset is 57) and is fatal within two to five years after diagnosis (Williams, et al. (1991) Mayo Clin. Proc., 66: 54-82). ALS affects approximately 30,000 Americans with nearly 8,000 deaths reported in the US each year. ALS patients progressively lose all motor function - unable to walk, speak, or breathe on their own.

The cardinal feature of ALS is the loss of spinal motor neurons, which causes the muscles under their control to weaken and waste away leading to paralysis. ALS has both familial (5-10%) and sporadic forms and the familial forms have now been linked to several distinct genetic loci (Deng, et al. (1995) Hum. Mol. Genet., 4: 1113-16; Siddique, et al. (1995) Clin. Neurosci., 3: 338-47; Siddique, et al., (1997) J. Neural Transm. Suppl., 49: 219-33; Ben Hamida, et al. (1990) Brain, 113: 347-63; Yang, et al. (2001) Nat. Genet. 29: 160-65; Hadano, et al. (2001) Nat. Genet. 29: 166-73). About 15-20% of familial cases are due to mutations in the gene encoding Cu/Zn superoxide dismutase 1 (SOD1) (Siddique, et al. (1991) N. Engl. J. Med., 324: 1381-84; Rosen, et al. (1993) Nature, 362: 59-62).

Although the etiology of the disease is unknown, one theory is that neuronal cell death in ALS is the result of over-excitement of neuronal cells due to excess extracellular glutamate. Glutamate is a neurotransmitter that is released by glutaminergic neurons, and is taken up into glial cells where it is converted into glutamine by the enzyme glutamine synthetase, glutamine then re-enters the neurons and is hydrolyzed by glutaminase to form glutamate, thus replenishing the neurotransmitter pool. In a normal spinal cord and brain stem, the level of extracellular glutamate is kept at low micromolar levels in the extracellular fluid because glial cells, which function in part to support neurons, use the excitatory amino acid transporter type 2 (EAAT2) protein to absorb glutamate immediately. A deficiency in the normal EAAT2 protein in patients with ALS, was identified as being important in the pathology of the disease *(See e.g.*, Meyer et al. (1998) J. Neurol. Neurosurg. Psychiatry, 65: 594-596; Aoki et al. (1998) Ann. Neurol. 43: 645-653; Bristol et al. (1996) Ann Neurol. 39: 676-679). One explanation for the reduced levels of EAAT2 is that EAAT2 is spliced aberrantly (Lin et al. (1998) Neuron, 20: 589-602). The aberrant splicing produces a splice variant with a deletion of 45 to 107 amino acids located in the C-terminal region of the EAAT2 protein (Meyer et al. (1998) Neureosci Lett. 241: 68-70). Due to the lack of, or defectiveness of EAAT2, extracellular glutamate accumulates, causing neurons to fire continuously. The accumulation of glutamate has a toxic effect on neuronal cells because continual firing of the neurons leads to early cell death

Although a great deal is known about the pathology of ALS little is known about the pathogenesis of the sporadic form and about the causative properties of mutant SOD protein in familial ALS (Bruijn, et al. (1996) Neuropathol. Appl. Neurobiol., 22: 373-87; Bruijn, et al. (1998) Science 281: 1851-54). Many models have been speculated, including glutamate toxicity, hypoxia, oxidative stress, protein aggregates, neurofilament and mitochondrial dysfunction Cleveland, et al. (1995) Nature 378: 342-43; Cleveland, et al. Neurology, 47(4 Suppl. 2): S54-61, discussion S61-2(1996); Cleveland, (1999) Neuron, 24: 515-20; Cleveland, et al. (2001) Nat. Rev. Neurosci., 2: 806-19; Couillard-Despres, et al. (1998) Proc. Natl. Acad. Sci. USA, 95: 9626-30; Mitsumoto, (1997) Ann. Pharmacother., 31: 779-81; Skene, et al. (2001) Nat. Genet. 28: 107-8; Williamson, et al. (2000) Science, 288: 399).

Presently, there is no cure for ALS, nor is there a therapy that has been proven effective to prevent or reverse the course of the disease. Several drugs have recently been approved by the Food and Drug Administration (FDA). To date, attempts to treat ALPS have involved treating neuronal degeneration with long-chain fatty alcohols which have cytoprotective effects *(See* U.S. Pat. No. 5,135,956); or with a salt of pyruvic acid (*See* U.S. Pat. No. 5,395,822); and using a glutamine synthetase to block the glutamate cascade *(See* U.S. patent 5,906,976). For example, Riluzole™, a glutamate release inhibitor, has been approved in the U.S. for the treatment of ALS, and appears to extend the life of at least some patients with ALS. However, some reports have indicated that even though Riluzole™ therapy can prolong survival time, it does not appear to provide an improvement of muscular strength in the patients. Therefore, the effect of RiluzoleA^{™} is limited in that the therapy does not modify the quality of life for the patient (Borras-Blasco et al. (1998) Rev. Neurol., 27: 1021-1027).

### II. SOD and SOD Mutations

The invention has the effect of decreasing the SOD-1 protein (e.g., mutant SOD-1 protein) in cells by reducing or eliminating the expression of the protein with a pyrimethamine and their functional analogs. The SOD-1 gene is localized to chromosome 21q22.1. SOD-1 sequences are disclosed in PCT publication WO 94/19493 are oligonucleotide sequences encoding SOD-1 and generally claimed is the use of an antisense DNA homolog of a gene encoding SOD-1 in either mutant and wild-type forms in the preparation of a medicament for treating a patient with a disease. The nucleic acid sequence of human SOD-1 gene can be found at Genbank accession no. NM_000454. The-nucleotide sequence of human SOD-1 is also presented in SEQ ID NO: 1. The corresponding SOD-1 protein sequence is presented in SEQ ID NO: 2.

### III. Pyrimethamine and Its Functional Analogs

In one aspect, the invention pertains the the use of pyrimethamine and its functional analogs. Pyrimethamine is an antimalarial drug, that readily penetrates cells in the body and brain. Pyrimethamine has been used for the treatment of malaria, toxoplasmosis, and several other microbial infections (for review see Schweitzer, et al. (1990) FASEB J 4:2441-2452). The antimicrobial effect of pyrimethamine is a result of its inhibition of dihydrofolate reductase (DHFR), and enzymes involved in the folate synthesis pathway. The malaria parasite synthesizes folates de novo whereas the human host must obtain preformed folates and cannot synthesize folate. The inability of the parasite to utilize exogenous folates makes folate biosynthesis a good drug target. DHFR is an ubiquitious enzyme that participates in the recycling of folates by reducing dihydrofolate to tetrahydofolate. The tetrahydrofolate is then oxidized back to dihydrofolate as it participates in biosynthetic reactions (e.g.., thymidylate synthase). Inhibiting DHFR will prevent the formation of thymidylate and lead to an arrest in DNA synthesis and subsequent parasite death. Pyrimethamine is the most common DHFR inhibitor used as antimalarials. Other DHFR inhibitors include, but are not limited to, sulfadoxine, trimethoprim, sulfadiazine, trimethoprim, and sulfamethoxazole.

By the use of the invention SOD-1 expression is lowered. Pyrimethamine is a potent inhibitor of SOD-1 expression in the HeLa cell and in the mouse Neuro2A cell lines as shown in the Examples. The mechanism of action for reduction of SOD-1 is not known at this time, but levels of both the protein and mRNA coding for SOD-1 are dose-dependently reduced. Pyrimethamine, however, does not act via dihydrofolate reductase inhibition, because its effects could not be prevented or reversed using folinic acid (the enzymatic product of DHFR). Furthermore, methotrexate, a potent DHFR inhibitor with an unrelated chemical structure, did not reduce SOD-1 protein in the HeLa cell. Finally, pyrimethamine is very weak inhibitor of human DHFR with an EC₅₀ > 70 µM. (Schweitzer *et al.*, (1990) *Supra*). Pyrimethamine has been documented to act on centromeric DNA and most likely inhibits transcription of the SOD-1 gene by an action on a transcription factor or less likely by a direct action on the genomic DNA itself.

While not required to provide a mechanism, it is believed that for inhibition of SOD-1 expression, pyrimethamine and its functional analogs putatively acts to reduce the expression of human SOD1 via an as yet unidentified nuclear receptor. Briefly, pyrimethamine binds with high affinity to the receptor protein, activating it and causing it to dimerize with another activated receptor. The dimerized receptors are transported into the cell's nucleus, or are already in the nucleus and bound to genomic DNA 5' to the start codon of hSOD1. The transported receptors bind to a stretch of palindromic DNA in the promoter region of the SOD-1 gene where the receptor-ligand-DNA complexes exert steric hindrance to prevent the initiation of transcription. Thus, less RNA coding for SOD1 is made, and consequently less protein is made. Reductions in the amount of mutant SOD1 protein produced would then prevent its neurotoxic effects and ameliorate ALS disease progression.

According to the invention, a pyrimethamine or a functional analog thereof is used. The pyrimethamine pharmacophore as shown in formula I.

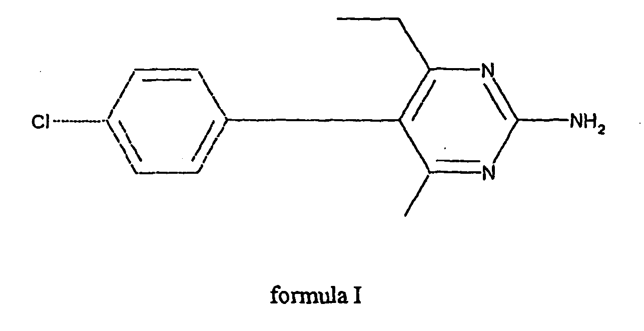

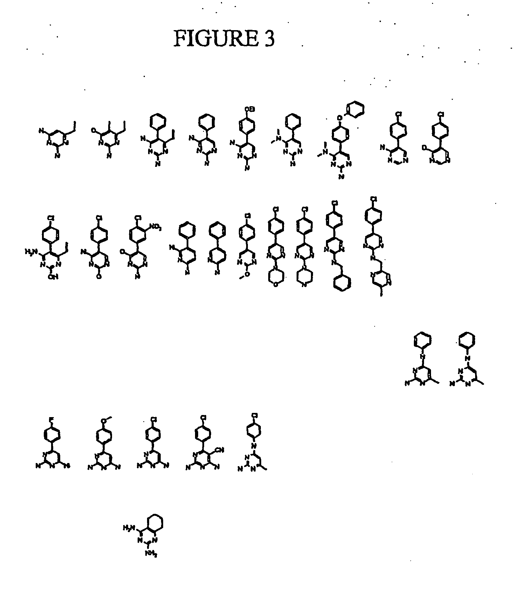

The structure-activity relationship (SAR) within pyrimethamine series of compounds can be established with a group of the representative 30 compounds shown in Figure 3. A systematic SAR study may demonstrate that these compounds do act by a specific cell target. Some of these compounds are commercially available, while others have been synthesized using standard organic chemistry methods. The design of the compounds is based on the lead molecule pyrimethamine may be broken into two main structural features and modified separately. The two main structural features are (1) the benzene ring, and (2) the pyrimidine ring. A number of analogues or derivatives have been designed, as described below:

### (1) Modification optimization of the benzene ring

The benzene ring of formula I can be modifi ed in a number of different ways. In one embodiment, by replacing R in formula II with a alkyl group selected from the group consisting of Me, Et, and the like.

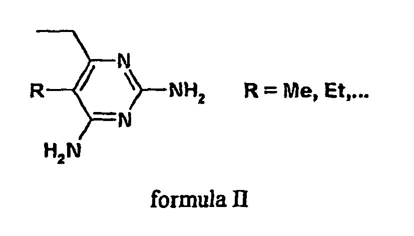

In another embodiment, by optimizing substituents at the benzene ring with respect to the position and the nature of the substituents. This may be performed by methyl scanning of the benzene ring at various positions as shown in formula III.

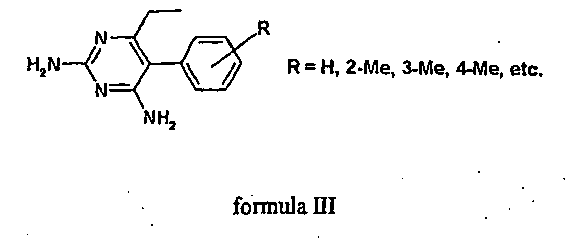

### (2) Modification of the pyrimidine ring:

The pyrimidine ring can be modified alone or in combination with the modifications to the benzene ring. In one embodiment, the pyrimidine ring is modified by removing or replacing the alkyl (R) group of formula V with other functional groups selected from the group consisting of H, Me, Isopropyl (iPr) or the like.

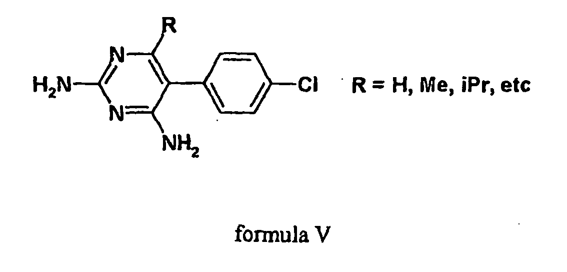

In another embodiment, the para-amino group (with respect to the benzene ring) can be removed or replaced with oxygen as shown in formula VI

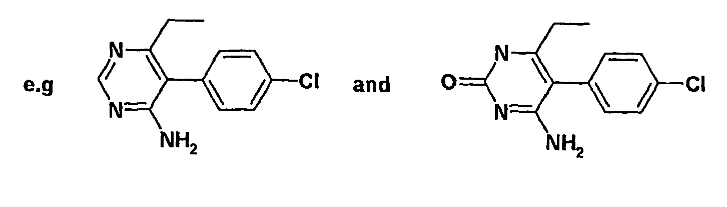

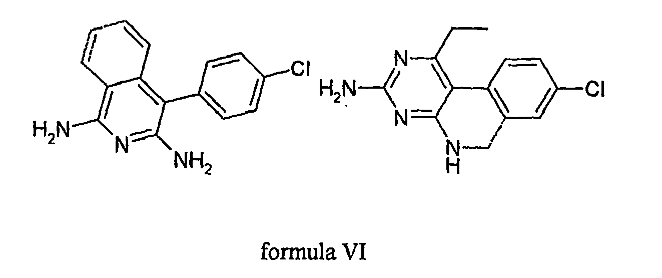

In another embodiment, the ortho-amino group (with respect to the benzene ring) can be removed or replaced with oxygen as shown in formula VII

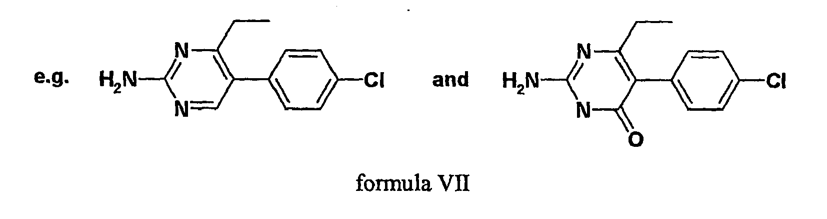

In another embodiment, both of the primary amine groups can be eliminated from the molecule as shown in formula VIII

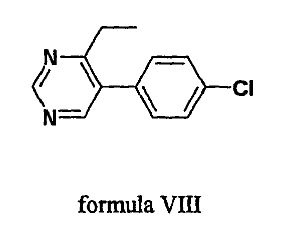

The compounds all contain the pharmachophoric features that are present in the lead structure shown in Formula I. These modifications are made to retain or improve the potency, oral activity and the drug property of the molecule. In other embodiments entirely new compounds are synthesized using standard organic synthetic chemistry. These new compounds retain the original pharmachophoric features. Analogs of the new compounds can also be synthesized. In one embodiment, a structure that removes rotational freedom between the two rings can be synthesized, as shown in formula XII.

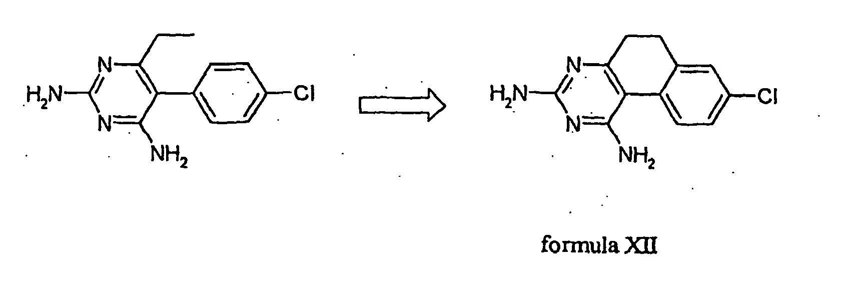

In another embodiment, a structure that ties up the two rings at the ortho-amino group can be synthesized, as shown in formula XIII.

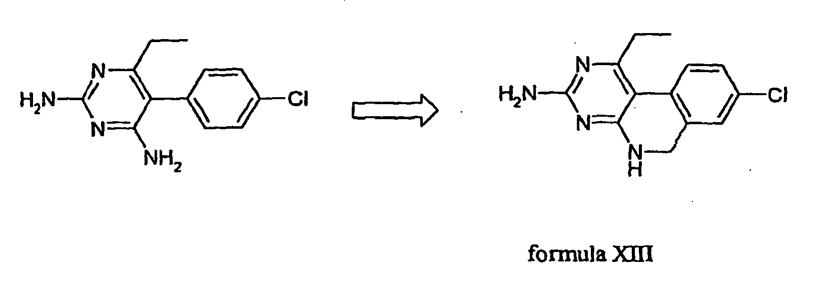

In other embodiments, the structures shown above and in Figure 3 and their analogs can be synthesized.

Some of these structures may be commercially available from comprehensive chemicals databases such as Available Chemical Directory. However, many of the structures are novel an unavailable on the databases. The synthesis of those compounds that are not commercially available can be carried out by well established synthetic methodologies. There are many synthesis papers and patents that describe the synthesis of all of the desired analogues. (See e.g., U.S. 3,940,393; Sardarian, et al., (2003) Org. Biomol. Chem. 21: 960-964; Ross, et al., (1976) J. Med. Chem. 19: 723-725).

### IV. Modulation of Neurodegenerative Disorders Using Pharmacological Agents

The role of the nuclear receptor in the neurodegenerative diseases such as ALS, and modulation of the pathway associated with the nuclear receptor maybe a target of a clinical investigation in ALS or other neurodegenerative disease. The data shown in the Examples section indicate that the pyrimethamine and its analogs play a role in decreasing the expression of SOD-1.

The SOD1 G93A (high copy) mouse model for ALS is a suitable mouse that carries 23 copies of the human G93A SOD mutation and is driven by the endogenous promoter. Survival in the mouse is copy dependent. The high copy G93A has a median survival of around 128 days. High molecular weight complexes of mutant SOD protein are seen in the spinal cord beginning around day 30. At day 60 reactive astrocytosis (GFAP reactive) are observed; activated microglia are observed from day 90 onwards. Studies by Gurney *et al*. showed that at day 90 reactive astrocytosis loses statistical significance while microglial activation is significantly elevated and continues to be elevated through the end stage of the disease (*See* Gurney, et al. (1996) Ann. Neurol., 39: 147-5739).

Many drugs that have shown efficacy in this model have moved forward into human clinical trials. Experience with riluzole, the only approved drug in the treatment of ALS, indicates that the mouse ALS model is a good predictor of clinical efficacy. Other drugs such as Creatine, Celebrex, Co-enzyme Q10, and Minocycline are under clinical evaluation based on studies in this model.

### V. Delivery of the Nuclear Receptor Modulating Pharmacological Agents

The pyrimethamine or a functional analog thereof for use according to the present invention can be incorporated into pharmaceutical compositions suitable for administration to a subject. Typically, the pharmaceutical composition comprises pyrimethamine or a functional analog therof and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the pharmacological agent

The pharmaceutical compositions may be in a variety of forms. These include, for example, liquid, semi-solid and solid dosage forms, such as liquid solutions (*e.g.*, injectable and infusible solutions), dispersions or suspensions, tablets, pills, powders, liposomes and suppositories. The preferred form depends on the intended mode of administration and therapeutic application. The preferred mode of administration is parenteral (*e.g*., intravenous, subcutaneous, intraperitoneal, intramuscular). In a preferred embodiment, the pharmacological agent is administered by an intraperitoneal injection.

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, (see, for example, Langer, Science 249, 1527 (1990) and Hanes, Advanced Drug Delivery Reviews 28, 97-119 (1997). The agents of this invention can also be administered in the form of a depot injection or implant preparation which can be formulated in such a manner as to permit a sustained or pulsatile release of the active ingredient. The depot injection or implant preparation can, for example, comprise one or more of the pyrimethamine compounds or functional analogs, or comprise a combination of different agents (e.g., pyrimethamine and norethindrone).

The pharmaceutical compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (*i.e.*, the pharmacological agent) in the required amount in an appropriate solvent with one or a combination of ingredients enumerated above, as required, followed by filtered sterilization.

Generally, dispersions are prepared by incorporating the active compound into a sterile vehicle that contains a basic dispersion medium and the required other ingredients from those enumerated above. In the case of sterile, lyophilized powders for the preparation of sterile injectable solutions, the preferred methods of preparation are vacuum drying and spray-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example monostearate salts and gelatin.

The pyrimethamine or a functional analog thereof for use according to the invention can be administered by a variety of methods known in the art. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. (*See, e.g.,* Sustained and Controlled Release Drug Delivery Systems, J.R Robinson, ed., Marcel Dekker, Inc., New York,1978; U. S. Patent Nos. 6,333,051 to Kabanov et al.*,* and 6,387,406 to Kabanov et al.).

In certain embodiments, the pyrimethamine or a functional analog thereof for use according to the invention may be orally administered, for example, with an inert diluent or an assimilable edible carrier. The above compound (and other ingredients, if desired) may also be enclosed in a hard or soft shell gelatin capsule, compressed into tablets, or incorporated directly into the subject's diet. For oral therapeutic administration, the compounds for use according to the invention may be incorporated with excipients and used in the form of ingestible tablets, buccal tablets, troches, capsules, elixirs, suspensions, syrups, wafers, and the like. To administer a compound for use according to the invention by other than parenteral administration, it may be necessary to coat the compound with, or co-administer the compound with, a material to prevent its inactivation.

In certain embodiments, the pyrimethamine or a functional analog thereof for use according to the invention can be administered in a liquid form. Said compounds should be soluble in a variety of solvents, such as for example, methanol, ethanol, and isopropanol. A variety of methods are known in the art to improve the solubility of a pharmacological agent in water and other aqueous solutions. For example, U.S. Patent No. 6,008,192 to Al-Razzak et al. teaches a hydrophilic binary system comprising a hydrophilic phase any surfactant, or mixture of surfactants, for improving the administration of compounds.

Supplementary active compounds can also be incorporated into the compositions. In certain embodiments, the pyrimethamine or a functional analog thereof for use according to the invention can be co-formulated with and/or co-administered with one or more additional therapeutic agents that are useful for improving the pharmacokinetics of the pharmacological agent. A variety of methods are known in the art to improve the pharmacokinetics of the use of the present invention. (See e.g., U.S. Patent No. 6,037,157 to Norbeck et al.)

Other methods of improving the pharmacokinetics of pharmacological agents have been disclosed, for example, in U.S. Patent Nos. 6,342,250 to Masters, 6,333,051 to Kabanov et al.*,* 6,395,300 to Straub et al., 6,387,406 to Kabanov et al.*,* and 6,299,900 to Reed et al. Masters discloses a drug delivery device and method for the controlled release of pharmacologically active agents. The drug delivery device disclosed by Masters is a film comprising one or more biodegradable polymeric materials, one or more biocompatible solvents, and one or more pharmacologically active agents dispersed uniformed throughout the film. In U.S. Patent No. 6,333,051, Kabanov et al. disclose a copolymer networking having at least one cross-linked polyamine polymer fragment, at least one nonionic water-soluble polymer fragment, and at least one suitable biological agent, including a pharmacological agent According to the teachings of this patent, this network, referred to as a nanogel network, improves the therapeutic effect of the pharmacological agent by decreasing side effects and increasing therapeutic action. In another patent, U.S. Patent No. 6,387,406, Kabanov et al. also disclose another composition for improving the oral delivery of numerous pharmacological agents.

Other methods for improving the delivery and administration of the pyrimethamine or a functional analog thereof for use according to the invention include means for improving the ability of said compounds to cross membranes, and in particular, to cross the blood-brain barrier. In one embodiment, the above compounds for use according to the invention can be modified to improve its ability to cross the blood-brain barrier, and in an alternative embodiment, the above compounds for use according to the invention can be co-administered with an additional agent, such as for example, an anti-fungal compound, that improves their ability to cross the blood-brain barrier. Alternatively, precise delivery of the above compounds for use according to the invention into specific sites of the brain, can be conducted using stereotactic microinjection techniques. For example, the subject being treated can be placed within a stereotactic frame base (MRI-compatible) and then imaged using high resolution MRI to determine the three-dimensional positioning of the particular region to be treated. The MRI images can then be transferred to a compute having the appropriate stereotactic software, and a number of images are used to determine a target site and trajectory for pharmacological agent microinjection. The software translates the trajectory into three-dimensional coordinates that are precisely registered for the stereotactic frame. In the case of intracranial delivery, the skull will be exposed, burr holes will be drilled above the entry site, and the stereotactic apparatus used to position the needle and unsure implantation at a predetermined depth. The compounds for use according to the invention can be delivered to regions, such as the cells of the spinal cord, brainstem, or brain that are associated with the disease or disorder. For example, target regions can include the medulla, pons, and midbrain, cerebellum, diencephalon (e.g., thalamus; hypothalamus), telencephalon (e.g., corpus stratium, cerebral cortex, or within the cortex, the occipital, temporal, parietal or frontal lobes), or combinations thereof.

The compounds for use according to the invention can be used alone or in combination to treat neurodegenerative disorders. For example, said compounds can be used in conjunction with other existing nuclear receptor modulators, for example, to produce an additive or synergistic effect. Likewise, said compounds can be used alone or in combination with an additional agent, *e.g.*, an agent which imparts a beneficial attribute to the therapeutic composition, *e.g.*, an agent which effects the viscosity of the composition. The combination can also include more than one additional agent, *e.g.*, two or three additional agents if the combination is such that the formed composition can perform its intended function. In some embodiments, the invention includes a pyrimethamine or functional analog thereof for use according to the invention,
together with for example, at least one progesterone related compound, such as norethindrone, or at least one estrogen related compound, such as estradiol. For descriptions of these compounds and admininstration, see co-pending applications entitled "Modulation ofNeurodegenerative Diseases through the Progesterone Receptor" and "Modulation of Neurodegenerative Diseases through the Estrogen Receptor" filed March 1, 2006.

The compounds for use according to the present invention can be conjugated with pharmaceutically acceptable acid salts to facilitate their long storage and dosing as aqueous solutions. For example, the salt can be derived from a pharmaceutically acceptable acid (e.g., HCI) with or without the use of a pharmaceutically acceptable carrier (e. g., water). Such salts can be derived from either inorganic or organic acids, including for example hydrochloric, hydrobromic, acetic, citric, fumaric, maleic, benzenesulfonic, and ascorbic acids. The pharmaceutical compositions obtained by the combination of the carrier and the salt will generally be used in a dosage necessary to elicit the desired biological effect. This includes its use in a therapeutically effective amount or in a lesser amount when used in combination with other biologically active agents.

The pharmaceutical compositions relating to the use of the invention may include a "therapeutically effective amount" or a "prophylactically effective amount" of a pyrimethamine or a functional analog thereof for use according to the invention. A "therapeutically effective amount" refers to an amount effetive, at dosages and for periods of time necessary, to achieve the desired therapeutic result. A therapeutically effective amount of the compounds for use according to the invention may vary according to factors such as the disease state, age, sex, and weight of the individual, and the ability of the pharmacological agent to elicit a desired response in the individual. A therapeutically effective amount is also one in which any toxic or detrimental effects of the pharmacological agent are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective_amount will be less than the therapeutical effective amount.

Dosage regimens within the use of the present invention may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form for the use according to the invention herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms for use according to the invention is dictated by and directly dependent on (a) the unique characteristics of the active compound and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of, e.g., pyrimethamine within the use of the invention is between 5 mg/day to about 200 mg/day administered to a subject, or group of subjects, preferably about 10 mg/day to about 150 mg/day, more preferably about 5 mg/day to about 20 mg/day, and most preferably about 3 mg/day to 10 mg/day. Preferably, administration of a therapeutically effective amount of, e.g., pyrimethamine within the use of the invention results in a concentration of pharmacological agent in the bloodstream in the range of 1 nanomolar (nM) to 100 millimolar (mM) concentration. For example, a concentration range of about 100nM to about 10mM, about, 1nM to about 1mM, about 1nM to about 100 micromolar (µM), about 1µM to about 500µM, about 1µM to about 200µM, or about 10µM to about 50µM. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions within the use of the invention, and that dosage ranges set forth herein are exemplary only and are not intended to limit the scope or practice of the use of the invention.

### Examples

### Example 1: Materials and methods:

### (i) Cell Culture

The human cervical carcinoma derived HeLa cell line (ATCC) was found to express SOD-1 protein and mRNA and was used as the model system to identify compounds that inhibit SOD-1 expression. Briefly, cells were maintained in Dulbecco's Minimal Essential Medium, with high glucose, supplemented with glutamine, 4 mM, certified fetal bovine serum, 10%, and penicillin, streptomycin, and nystatin (all from Invitrogen). Incubation conditions were 37 degrees and 99% relative humidity, with CO₂ at 5%. Cultures were passaged when they reached 90% confluence. For pharmacological experiments, cells were plated into sterile tissue culture treated 96 well plates at a density of 3,500 cells/well in 150 µl medium

### (ii) Drugs:

All compounds were dissolved in 100% DMSO, at a stock concentration of 10 mM. Drugs were obtained from Microsource Discovery or from Sigma Aldrich.

### (iii) Experimental Protocol:

After plating and 6 hours for attachment, drugs were added to the medium in a concentration of 10 µM. Following 72 hours of incubation with the drugs, the cells were photographed at 100X using an inverted microscope and digital camera, so that cytotoxicity could be evaluated. After photodocumentation, the medium was removed and the cells were washed once with phosphate buffered saline, and then 50 µ**l** molecular biology grade water containing a protease inhibitor cocktail was added. After 10 min incubation, the plates were placed in -80 degrees to induce complete lysis. Plates were then thawed and 25 µl was transferred from each well into a maxsorp ELISA plate coated with anti-human SOD-1 antibody, which contained 75 µl phosphate buffered saline. A second antibody pair (a polyclonal anti-SOD-1/HRP conjugated goat anti-rabbit) was then added to the well, and incubation was conducted for 1 hour at room temperature. At the conclusion of the incubation, the plate was washed three times (wash buffer from KPL Inc.) and Sure Blue Reserve HRP Substrate was added. Following a 5-10 min incubation, the reaction (which had turned blue to varying degrees) was stopped by the addition of a stop reagent (KPL). The plate was then shaken gently for 5 seconds and the absorbance at 450 nm read on a Tecan Plate reader. Absorbance from each sample were compared to standard curve of purified recombinant human SOD-1 assayed on the same ELISA plate, and SOD-1 immunoreactivity (ng/ml) was estimated by comparison with the standard curve.

### (ii) Bradford Protein Assay:

To determine if decrements found in the SOD-1 assay were simply the result of cytotoxic effects of the drug treatment, total protein was determined for each well. While the ELISA incubation was ongoing, 10 µl of the remaining lysate was removed from each well and placed into another empty plate, and BioRad Bradford reagent (100 µl) was added to the protein. After a 15 min incubation at room temperature the plate was shaken gently for 5 seconds and the absorbance was read at 595 nm in a Tecan Sunrise plate reader. Protein concentrations in each well were thus determined by comparison with protein standards that were run on the same plate.

### (v) Quantitative RT-PCR:

HeLa cells at 3500 cells/well in a 96 well plate were treated with a compound of the present invention for 72 h as above and then cells were lysed and total RNA extracted using the Gentra RNA extraction protocol and reagents. The purified RNA was then used as the template in a reverse transcription reaction using Superscript III MMLV Transcriptase primed with oligoDT. A PCR reaction was performed on the resultant cDNA to amplify the cDNA corresponding to human SOD-1, human TATA-box binding protein, and human Beta-2 microglobulin. The PCR reactions were run in separate tubes for 20, 25, and 30 cycles and the amplicons were then run on a 2% agarose gel containing ethidium bromide. The fluorescence emitted by the ethidium bromine stained bands following stimulation by a UV light source was captured using a digital camera. The digitized images were analyzed using ImageJ (NIH) and the bands for SOD-1 were compared with the bands for TATA-box binding protein and Beta2 Microglobulin (these housekeeping genes were unaffected by the drugs) while in the linear range of cycles, 25 cycles under these conditions, for increases or decreases relative to controls.

### (vi) GeneChip Experiments:

Total cellular mRNA was prepared from HeLa cells with or without treatment using a Qiagen RNA mini kit followed by oligotex mRNA mini kit. Double-stranded cDNAs were synthesized from 2 µg total mRNA using the Superscript Choice System for cDNA synthesis (Invitrogen) with the T7-(dT)24 primer following the manufacturer's recommendations. cDNAs were cleaned up by phase lock gel (PLG) phenol/chloroform extraction and concentrated by ethanol precipitation. Biotin-labeled cRNA was synthesized from cDNA by in vitro transcription using the Bioarray HighYield RNA transcript Labeling Kit (Affymetrix) following vendor's recommendation. In vitro transcription products were cleaned up using RNeasy spin columns (Qiagen) and fragmented by metal-induced hydrolysis in fragmentation buffer (40 mM Tris-acetate, pH 8.1, 100 mM KOAc, 30 mM MgOAc). Fragmented cRNA was then subjected to Affymetrix GeneChip sets in hybridization buffer (100 mM MES, 1M NaCl, 20 mM EDTA, 0.01% Tween-20). GeneChip images were analyzed with Affymetrix Microarray Suite V5.0 and Affymetrix Data Mining Tool V3.0. Signal intensities of all probe sets were scaled to a target value of 150. Results of Detection Call, Change Call and Signal Log Ratio were obtained by applying the default parameters to statistical algorithms for both absolute and comparison analyses.

### (vii) Western blotting.

Animals were overdosed with sodium pentobarbital (250 mg/kg, i.p.). Spinal cords were dissected and homogenized in 20 mM Tris-HCl, pH 7.5, 2 mM DTT, 0.1 mg of leupeptin, 1 mM EDTA, and 1 mM EGTA. The homogenate was then centrifuged at 14,000 × g to pellet debris. Protein concentration was measured using the BCA protein assay (Pierce, Rockford, IL). Protein (25 µg) from each sample was run on a 4-20% Tris-glycine gel (Invitrogen, San Diego, CA). After transfer, membranes were washed in PBS, followed by overnight incubation in blocking buffer (0.2% I-block (Applied Biosystems, Foster City, CA), PBS, and 0.1 % Tween 20). The membrane was then probed with a polyclonal rabbit anti-bovine SOD1 (Sigma) antibody at 1:4000 dilution. After several washes, membranes were incubated with an alkaline phosphatase-conjugated secondary antibody (1:5000 in blocking buffer), and the immunoreactive signals were visualized using an enhanced chemiluminescent reagent, CDP Star (Western Star kit; Tropix). After exposure, films were scanned and then imported into NIH Image for quantitation of band density.

### Example 2: Testing the Effects of an Antimalarial Agent

This example describes how to examine the *in vitro* effects of the antimalarial drug, pyrimethamine, on SOD-1 activity. The human cervical carcinoma derived HeLa cell line (ATCC) were cultured in Dulbecco's Minimal Essential Medium, with high glucose, supplemented with glutamine, 4 mM, certified fetal bovine serum, 10%, and penicillin, streptomycin, and nystatin (all from Invitrogen). Incubation conditions were 37°C and 99% relative humidity, with CO₂ at 5%. Cultures were passaged when they reached 90% confluence. For pharmacological experiments, cells were plated into sterile tissue culture treated 96 well plates at a density of 3,500 cells/well in 150 µl medium

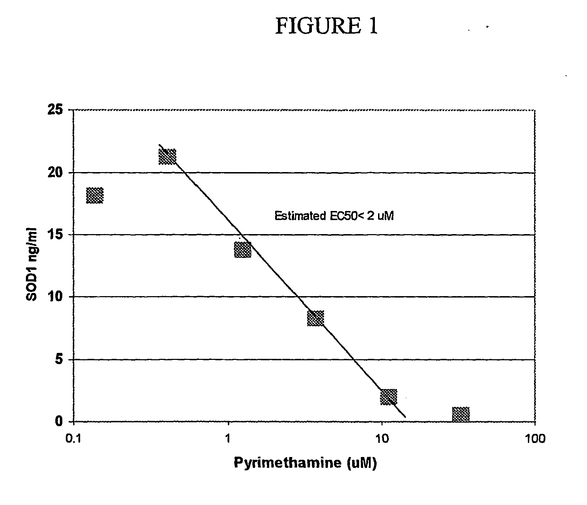

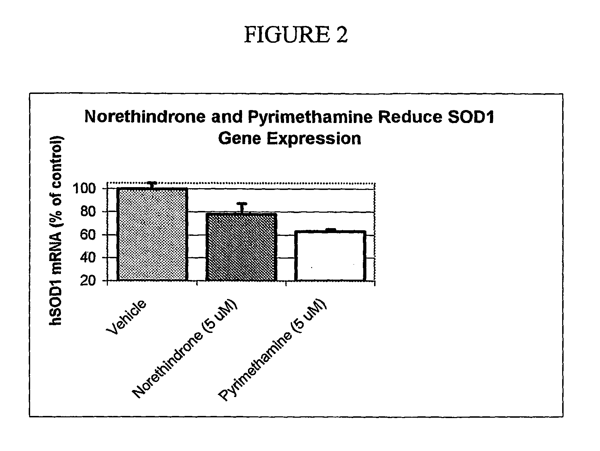

Following 72 hours of incubation with the pyrimethamine, the cells were photographed and processed as described in Example 1 (iii). The total protein of the lysates was determined by Bradford assay as described in Example 1 (iv). The results of this study are shown in Figure 1. These results show that pyrimethamine added to culture medium of HeLa cells 72 hours before harvest significantly reduced the levels of SOD-1 protein, while total protein levels were unaffected. This reduction was dose related and maximal by 10 µM, with an IC₅₀ of less than 3 µM. Figure 2 shows that both norethindrone and pyrimethamine (5 µM) caused a dose-related decrease in hSOD-1 mRNA in HeLa cells following 72 h treatment.

Alpha-synuclein has been implicated in neurodegenerative disorders characterized by Lewy body inclusions such as Parkinson's disease (PD) and dementia with Lewy bodies. Lewy body-like inclusions have also been observed in spinal neurons of patients with amyotrophic lateral sclerosis (ALS) and reports suggest possible alpha-synuclein abnormalities in ALS patients alpha-Synuclein is a ubiquitous protein that shares significant physical and functional homology to the protein chaperone, 14-3--3, and is particularly abundant in the brain (OstrerovaN. et al., J. Neurosci., 19:5782 (1990)). An increased rate of alpha-synuclein aggregation might contribute to the mechanisms ofneurodegeneration in Lewy body diseases. Studies on transgenic animals also suggest that aggregation of alpha-synuclein is harmful to neurons. It was reported that dopaminergic dysfunction occurred in transgenic mice expressing wild type human alpha-synuclein (Masliah, E., et at., Science, 287:1265-1269 (2000)) and that Drosophila over-expressing alpha-synuclein exhibited dopaminergic dysfunction and dopaminergic neuronal death associated with development of alpha-synuclein aggregates (Feany, M B, et al., Nature 404:394-8 (2000)). Evidence suggests that neurons with dopamine develop alpha-synuclein aggregates and degenerate as these aggregates development.

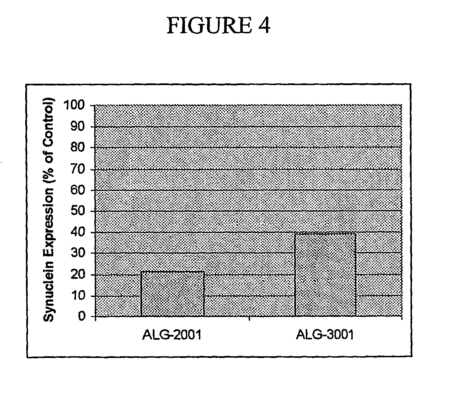

The results of the Genechip analysis shown in Figure 4 illustrates that pyrimethamine (ALG-2001) (3µM) and norethindrone (ALG-3001) (3µM) substantially decreased mRNA for alpha synuclein in HeLa cells following 4 days of treatment. Thus, the compounds of the present invention can slow neurodegeneration in Lewy body diseases. This illustrates a role for the compounds of the present invention in slowing the progression or ameliorating the effects of ALS and PD.

### Example 3: Testing the Effects of Pharmacological Agents In vivo

### (a) SOD-93A murine model

The effects of the pharmacological agents e.g., pyrimethamine, and analogs thereof described in Example 2 were tested *in vivo* in the SOD-93A murine model for ALS and a reduction in the SOD-1 levels was measured. The inhibition of RNA expression was monitored by isolated blood samples from the art recognized mouse model of ALS pre- and post introduction of the compound using standard RT-PCR techniques. The expression of the SOD-1 protein was determined using Western blot techniques with an anti-SOD-1 antibody from Sigma.

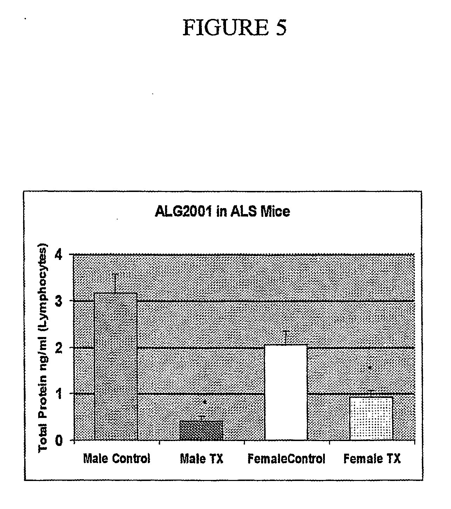

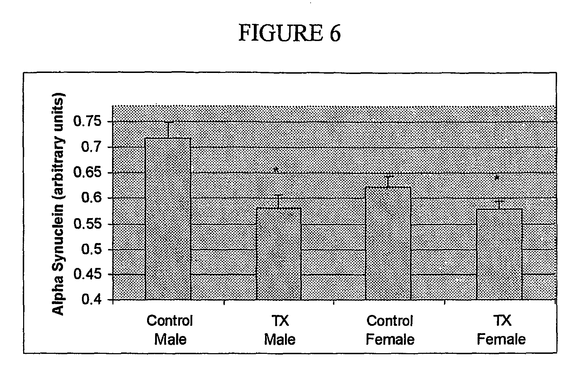

As shown in Figures 5 and 6, chronic treatment with pyrimethamine (10 mg/kg ip X 14 days) significantly (P< 0.05, n = 7) decreased SOD-1 protein and alpha synuclein in mouse lymphocytes. The results were show to be statistically significant using a student t-test analysis. The control was vehicle (saline).

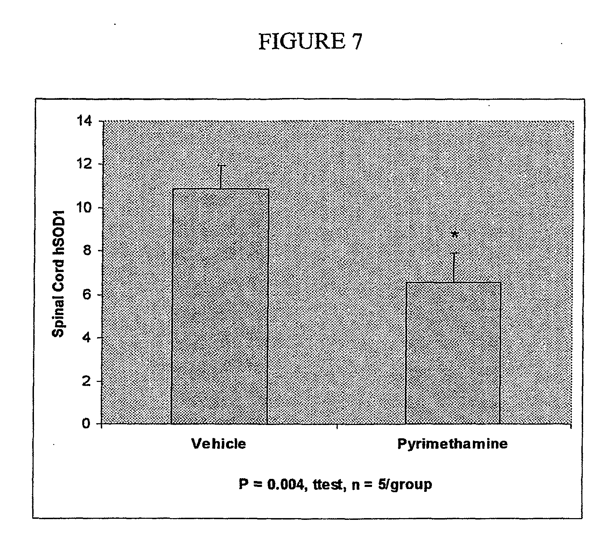

Chronic pyrimethamine (50 mg/kgld) significantly decreased spinal SOD-1 in G93A mice following 14 d treatment as shown in Figure 7. Pyrimethamine was administered orally for 14 days. Spinal cords were harvested and analyzed by Western blot analysis as described above.

### (b) Human familial ALS patient

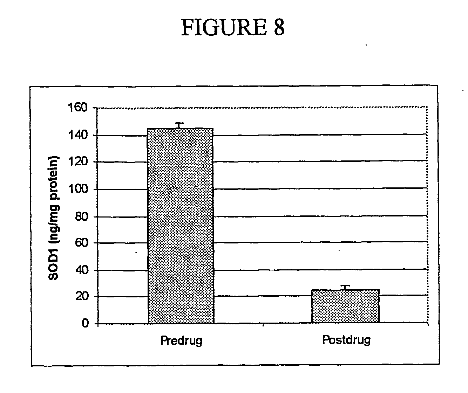

A 38 year old familial ALS patient volunteer showed a significant decrease in SOD-1 levels following oral treatment with pyrimethamine (100 mg/d for 30 days). Figure 8 shows the decreased lymphocyte SOD1 levels in the familial SOD1 patient following administration of the drug (post drug) compared to prior to treatment (predrug). Approximately 5-8 cc blood was collected from the patient. SOD-1 levels were analyzed by ELISA and Western blot analysis.

The *in vivo* effects can also be determined by monitoring the breathing of a subject by measuring the forced vital capacity (FVC) using a Renaissance Puritan Bennett Spirometer. The maximum inspiratory force (MIF) can also be measured using a hand held manometer.

### Example 4: Neurological Scoring

The effects of the nuclear receptor modulating pharmacological agents being pyrimethamine or a functional analog thereof can also be determined by a neurological score recorded on a 4-point scale:

| | |
|---|---|
| 0 = | Normal reflex on the hind limbs (animal will splay its hind limbs when lifted by its tail) |
| 1 = | Abnormal reflex (Lack of splaying of hind limbs when animal is lifted by the tail). |
| 2 = | Abnormal reflex and visible evidence of paralysis |
| 3 = | Lack of reflex and total paralysis of hind limbs. |
| 4 = | Inability to right themselves when placed on the sides in 30 seconds or found dead. The animals are sacrificed at this stage if alive. |

Statistical analysis on the neurological score, body weight and survival can be performed by utilizing ANOVA, Kaplan Meier, t-test, Cox's proportional hazards regression model, log-logistic and parametric methods and mixed linear model methods. All statistical analysis was performed using standard procedures known in the art.

## Claims

1. Use of a pyrimethamine or functional analog thereof for the manufacture of a medicament for preventing the development of symptoms, or ameliorating the symptoms or progression of SOD-1-mediated amyotrophic lateral sclerosis (ALS).

2. The use of claim 1, wherein the functional analog is pyrimethamine with at least one modification in the benzene ring.

3. The use of claim 1, wherein the functional analog is pyrimethamine with at least one modification in the pyrimidine ring.

4. A pyrimethamine or a functional analog thereof for use in a method for preventing the development of symptoms, or ameliorating the symptoms or progression of SOD-1-mediated amyotrophic lateral sclerosis (ALS).

5. A pyrimethamine or a functional analog thereof for use according to claim 4, wherein the functional analog is pyrimethamine with at least one modification in the benzene ring.

6. A pyrimethamine or a functional analog thereof for use according to claim 1, wherein the functional analog is pyrimethamine with at least one modification in the pyrimidine ring.

## Patentansprüche

1. Verwendung eines Pyrimethamin oder eines funktionellen Analogon davon für die Herstellung eines Medikaments zur Prävention der Entwicklung von Symptomen oder zur Verbesserung der Symptome oder der Progression von SOD-1-vermittelter amyotropher Lateralsklerose (ALS).

2. Verwendung gemäß Anspruch 1, wobei das funktionelle Analogon Pyrimethamin mit wenigstens einer Modifikation im Benzolring ist.

3. Verwendung gemäß Anspruch 1, wobei das funktionelle Analogon Pyrimethamin mit wenigstens einer Modifikation im Pyrimidinring ist.

4. Pyrimethamin oder ein funktionelles Analogon davon für eine Verwendung in einem Verfahren zur Prävention der Entwicklung von Symptomen oder zur Verbesserung der Symptome oder der Progression von SOD-1-vermittelter amyotropher Lateralsklerose (ALS).

5. Pyrimethamin oder ein funktionelles Analogon davon für eine Verwendung gemäß Anspruch 4, wobei das funktionelle Analogon Pyrimethamin mit wenigstens einer Modifikation im Benzolring ist.

6. Pyrimethamin oder ein funktionelles Analogon davon für eine Verwendung gemäß Anspruch 1, wobei das funktionelle Analogon Pyrimethamin mit wenigstens einer Modifikation im Pyrimidinring ist.

## Revendications

1. Utilisation de la pyriméthamine ou d'un analogue fonctionnel de celle-ci pour la fabrication d'un médicament destiné à la prévention du développement des symptômes ou à l'amélioration des symptômes ou du progrès de la sclérose latérale amyotrophique (SLA) causée par la SOD1.

2. Utilisation selon la revendication 1, dans laquelle l'analogue fonctionnel est la pyriméthamine avec au moins une modification sur le noyau benzénique.

3. Utilisation selon la revendication 1, dans laquelle l'analogue fonctionnel est la pyriméthamine avec au moins une modification sur le noyau pyrimidique.

4. Pyriméthamine ou un analogue fonctionnel de celle-ci pour l'utilisation dans une méthode pour la prévention du développement des symptômes ou l'amélioration des symptômes ou du progrès de la sclérose latérale amyotrophique (SLA) causée par la SOD1.

5. Pyriméthamine ou un analogue fonctionnel de celle-ci pour l'utilisation selon la revendication 4, l'analogue fonctionnel étant la pyriméthamine avec au moins une modification sur le noyau benzénique.

6. Pyriméthamine ou un analogue fonctionnel de celui-ci pour l'utilisation selon la revendication 1, l'analogue fonctionnel étant la pyriméthamine avec au moins une modification sur le noyau pyrimidique.


### SEQUENCE LISTING

<110> Scott, Sean
   Benjamin, Daniel
<120> Modulation of Neurodegenerative Diseases
<130> 106792-0009
<140> Not Yet Assigned
   <141> 2006-03-01
<150> 60/658,505
   <151> 2005-03-04
<160> 2
<170> PatentIn version 3.2
<210> 1
   <211> 2288
   <212> DNA
   <213> Homo sapiens
<400> 1
   gtaccctgtt tacatcattt tgccattttc gcgtactgca accggcgggc cacgccgtga     60
   aaagaaggtt gttttctcca cagtttcggg gttctggacg tttcccggct gcggggcggg    120
   gggagtctcc ggcgcacgcg gccccttggc ccgccccagt cattcccggc cactcgcgac    180
   ccgaggctgc cgcagggggc gggctgagcg cgtgcgaggc cattggtttg gggccagagt    240
   gggcgaggcg cggaggtctg gcctataaag tagtcgcgga gacggggtgc tggtttgcgt    300
   cgtagtctcc tgcaggtctg gggtttccgt tgcagtcctc ggaaccagga cctcggcgtg    360
   gcctagcgag ttatggcgac gaaggccgtg tgcgtgctga agggcgacgg cccagtgcag    420
   ggcatcatca atttcgagca gaaggcaagg gctgggaccg ggaggcttgt gttgcgaggc    480
   cgctcccgac ccgctcgtcc ccccgcgacc ctttgcatgg acgggtcgcc cgccagggct    540
   agagcagtta agcagcttgc tggaggttca ctggctagaa agtggtcagc ctgggattgc    600
   atggacggat ttttccactc ccaagtctgg ctgcttttta cttcactgtg aggggtaaag    660
   gtaaatcagc tgttttcttt gttcagaaac tctctccaac tttgcacttt tcttaaagga    720
   aagtaatgga ccagtgaagg tgtggggaag cattaaagga ctgactgaag gcctgcatgg    780
   attccatgtt catgagtttg gagataatac agcaggtggg tcataattta gctttttttt     840
   cttcttctta taaataggct gtaccagtgc aggtcctcac tttaatcctc tatccagaaa    900
   acacggtggg ccaaaggatg aagagaggta acaagatgct taactcttgt aatcaatggc    960
   gatacgtttc tggagttcat atggtatact acttgtaaat atgtgcctaa gataattccg 1020
   tgtttccccc acctttgctt ttgaacttgc tgactcatgt gaaaccctgc tcccaaatgc 1080
   tggaatgctt ttacttcctg ggcttaaagg aattgacaaa tgggcactta aaacgatttg 1140
   gttttgtagc atttgattga atatagaact aatacaagtg ccaaagggga actaatacag 1200
   gaaatgttca tgaacagtac tgtcaaccac tagcaaaatc aatcatcatt tgatgctttt 1260
   catataggca tgttggagac ttgggcaatg tgactgctga caaagatggt gtggccgatg 1320
   tgtctattga agattctgtg atctcactct caggagacca ttgcatcatt ggccgcacac 1380
   tggtggtaag ttttcataaa ggatatgcat aaaacttctt ctaacagtac agtcatgtat 1440
   ctttcacttt gattgttagt cgcgaattct aagatccaga taaactgtgt ttctgctttt 1500
   aaactactaa atattagtat atctctctac taggattaat gttatttttc taatattatg 1560
   aggttcttaa acatcttttg ggtattgttg ggaggaggta gtgattactt gacagcccaa 1620
   agttatcttc ttaaaatttt ttacaggtcc atgaaaaagc agatgacttg ggcaaaggtg 1680
   gaaatgaaga aagtacaaag acaggaaacg ctggaagtcg tttggcttgt ggtgtaattg 1740
   ggatcgccca ataaacattc ccttggatgt agtctgaggc cccttaactc atctgttatc 1800
   ctgctagctg tagaaatgta tcctgataaa cattaaacac tgtaatctta aaagtgtaat 1860
   tgtgtgactt tttcagagtt gctttaaagt acctgtagtg agaaactgat ttatgatcac 1920
   ttggaagatt tgtatagttt tataaaactc agttaaaatg tctgtttcaa tgacctgtat 1980
   tttgccagac ttaaatcaca gatgggtatt aaacttgtca gaatttcttt gtcattcaag 2040
   cctgtgaata aaaaccctgt atggcactta ttatgaggct attaaaagaa tccaaattca 2100
   aactaaatta gctctgatac ttatttatat aaacagcttc agtggaacag atttagtaat 2160
   actaacagtg atagcatttt attttgaaag tgttttgaga ccatcaaaat gcatacttta 2220
   aaacagcagg tcttttagct aaaactaaca caactctgct tagacaaata ggctgtcctt 2280
   tgaagctt 2288
<210> 2
   <211> 153
   <212> PRT
   <213> Homo sapiens
<400> 2
   Ala Thr Lys Ala Val Cys Val Leu Lys Gly Asp Gly Pro Val Gln Gly
   1               5                   10                  15
   Ile Ile Asn Phe Glu Gln Lys Glu Ser Asn Gly Pro Val Lys Val Trp
               20                  25                  30
   Gly Ser Ile Lys Gly Leu Thr Glu Gly Leu His Gly Phe His Val His
           35                  40                  45
   Glu Phe Gly Asp Asn Thr Ala Gly Cys Thr Ser Ala Gly Pro His Phe
       50                  55                  60
   Asn Pro Leu Ser Arg Lys His Gly Gly Pro Lys Asp Glu Glu Arg His
   65                  70                  75                  80
   Val Gly Asp Leu Gly Asn Val Thr Ala Asp Lys Asp Gly Val Ala Asp
                   85                  90                  95
   Val Ser Ile Glu Asp Ser Val Ile Ser Leu Ser Gly Asp His Cys Ile
               100                 105                 110
   Ile Gly Arg Thr Leu Val Val His Glu Lys Ala Asp Asp Leu Gly Lys
           115                 120                 125
   Gly Gly Asn Glu Glu Ser Thr Lys Thr Gly Asn Ala Gly Ser Arg Leu
       130                 135                 140
   Ala Cys Gly Val Ile Gly Ile Ala Gln
   145                 150